# EUROPÄISCHE PATENTANMELDUNG

(11) **EP 2 092 904 A1**
(43) Veröffentlichungstag der Anmeldung: **26.08.2009**
(21) Anmeldenummer: 08003236.0
(22) Anmeldetag: 22.02.2008
(51) Int. Cl.: A61B 17/80

(54) **Trochanterplatte**

(71) Anmelder: Prévôt, Bertrand, 22763 Hamburg (DE); Zlowodzki, Michal P. Dr. Med., Minneapolis MN 55403 (US); Bhandari, Mohit M.D., Hamilton ON L9C 6S8 (CA)
(72) Erfinder: Zlowodzki, Michael P., Minneapolis, MN 55403 (US)
(74) Vertreter: Meyer, Ludgerus

(57) **Zusammenfassung**

Die Erfindung betrifft eine Trochanterplatte zur Fixation eines Oberschenkelhalsknochens nach einer Fraktur mittels Knochenschrauben, wobei die Trochanterplatte (1) Bohrungen zur Aufnahme und Winkelfixierung der Knochenschrauben (4) aufweist. Erfindungsgemäß weist wenigstens eine der Bohrungen Mittel (20) zur befestigungsunabhängigen Ausrichtung einer Knochenschraube in einem begrenzten Winkelbereich auf. Die Schraubenköpfe (5) der als Spongiosaschrauben ausgebildeten Knochenschrauben (4) sind mittels Schraubkappen (8) in den Bohrungen der Trochanterplatte durch Klemmung gegen Bohrungsrand und Schraubkappe fixierbar. In Längsrichtung weist die Trochanterplatte (1) einen Ansatz (17) auf, der wenigstens eine Bohrung zur Aufnahme von Schrauben (18) mit oder ohne Winkelfixierung enthält, die in den Knochenschaft einschraubbar sind. Die Trochanterplatte (1) ist ferner entsprechend dem Oberflächenverlauf im Bereich des proximalen Endes eines Femurknochens vorgeformt.

## Beschreibung

Die Erfindung betrifft eine Trochanterplatte zur Fixation eines Oberschenkelhalsknochens nach einer Fraktur gemäß dem Oberbegriff des Anspruchs 1.

Der Oberschenkelhalsknochen ist im menschlichen Körper einer derjenigen Knochen, die, insbesondere bei alten Menschen, bei Stürzen oder Unfällen am häufigsten brechen. Die Bruchstelle befindet sich dabei zwischen dem Oberschenkelkopf und dem proximalen Ende des Femur. Oberschenkelkopf und Femur sind über den Oberschenkelhalsknochen in einem Winkel von etwa 135° (Corpus-Collum-Diaphysenwinkel, auch CCD-Winkel genannt), miteinander verbunden.

Zur Fixation eines gebrochenen Oberschenkelhalsknochens werden in der Regel Knochenschrauben verwendet, die von der Außenseite des Femur durch den Oberschenkelhalsknochen bis in den Oberschenkelkopf geführt werden. Die Längskräfte der Schrauben werden dabei durch den Knochen selbst aufgenommen. Das Einbringen der Schrauben hat mit hoher Präzision zu erfolgen. Ferner sind die Schrauben exakt auszurichten und ihre Köpfe sind an der Oberfläche des Knochens exakt festzulegen.

Gleichwohl ist es bekannt, dass in bis zu 30% der Fälle Nachoperationen erforderlich sind. Ferner existiert eine hohe Sterblichkeit von Patienten mit Oberschenkelhalsbrüchen.

Es sind ferner Knochenplatten bekannt, die der Fixierung von Knochenteilen dienen und die die Fragmente, die durch den Bruch getrennt sind, überbrücken und fixieren. Bei einem Oberschenkelhalsbruch ist es jedoch nicht möglich, die Knochenfragmente direkt mit einer Stützplatte zu verbinden.

Aus der WO 2004/045432 A1 ist eine Knochenplatte zur Abstützung von Schrauben bekannt, die der Verbindung zweier Bruchstücke des Oberschenkelhalsknochens dienen. Die Knochenplatte weist eine konvexe Oberseite, eine konkave Unterseite und wenigstens zwei Löcher zur Aufnahme von Knochenschrauben mit einem Kopf auf, wobei die Löcher derart ausgebildet sind, dass der Kopf der Knochenschraube darin in einem festen Winkel verankert werden kann. Als Option ist dort auch angegeben, eine der Schrauben als nicht winkelstabile Schraube einzusetzen.

Eine wesentliche Beschränkung dieser Platte liegt in der Tatsache, dass die winkelstabilen Schrauben mono-axial sind, d.h. nur in einem festen Winkel in der Platte verankerbar sind. Dies ist problematisch, da der Oberschenkelhalswinkel (CCD-Winkel) sehr variabel ist und der Oberschenkelhals an sich relativ schmal ist. Deswegen ist eine optimale Positionierung von mehreren Schrauben im Oberschenkelhals/Oberschenkelkopf, ohne dass Schrauben außerhalb des Oberschenkelhalses verlaufen, sehr schwierig, wenn man Patienten mit unterschiedlichen CCD-Winkeln behandelt.

Die DE 697 14 340 T2 bzw. EP 0 807 420 B1 zeigt eine Knochenbefestigungsvorrichtung für die Osteosynthese der Wirbelsäule, bei der polyaxiale Schrauben verwendet werden, die in einem variablen Winkel in die Knochensubstanz eingeschraubt werden können und deren Winkelstabilisierung durch Einschrauben einer Klemmkappe in den Kopfaufnahmebereich der Befestigungsvorrichtung erreichbar ist. Bei dieser Einrichtung können die Schrauben daher in einem begrenzten Winkelbereich variabel eingesetzt werden und erhalten ihre Winkelfixierung erst nach ihrer Festlegung durch eine Schraubkappe.

In der EP 0 988 833 B1 ist eine ähnliche Osteosyntheseplatte mit mehreren Knochenschrauben angegeben, bei der die Schraubkappe ein zusätzliches Innengewinde aufweist, durch das eine Erhöhung der Winkelstabilität der Knochenschrauben erzielt werden kann.

In der EP 1 153 577 A1 ist eine Knochenplatte angegeben, die offenbar ebenfalls als Osteosyntheseplatte mit mehreren Knochenschrauben ausgebildet ist, bei der durch eine spezielle flache Bauchform der Schraubenkappen eine sehr geringe Bauhöhe der Knochenplatte erzielbar ist. Allerdings verringert sich dadurch der verwendbare Winkelbereich der Knochenschrauben.

Schließlich ist es aus der US 5,484,439 bekannt, Knochenplatten mit einer winkelstabilen Hülse zu versehen, die eine in Längsrichtung der Führungshülse verschiebbare Schraube aufnehmen kann, die über die Fraktur eines Oberschenkelhalsknochens in den Oberschenkelkopf eingeschraubt werden kann.

Es hat sich herausgestellt, dass eine sichere und den Heilungsprozess fördernde Fixierung eines Oberschenkelhalsknochens mit winkelfixierten Schrauben nicht ausreichend ist. Dies liegt einerseits an der geringen Knochenqualität im Oberschenkelkopf, die es schwierig macht, Schrauben fest zu verankern. Der Oberschenkelhals ist außerdem relativ schmal und kann nur wenige Schrauben aufnehmen. Die anatomische Form des Femur ist außerdem sehr variabel, so dass eine Anpassung der Fixierungsvorrichtung erforderlich ist.

Die Erfindung kombiniert verschiedene für sich bekannte Elemente zu einem vorteilhaften System, das den anatomischen Gegebenheiten Rechnung trägt und die Struktur der Knochenfragmente berücksichtigt.

Der Erfindung liegt daher die Aufgabe zugrunde, eine Trochanterplatte bzw. ein System zur Fixation eines Oberschenkelhalsknochens anzugeben, die klein und anatomisch gut verträglich ausgebildet ist, eine präzise Fixierung der Fraktur ermöglicht und einfach und kostengünstig herstellbar ist.

Diese Aufgabe wird durch die in den Ansprüchen 1 und 12 angegebene Erfindung gelöst. Vorteilhafte Weiterbildungen der Erfindung sind in den jeweiligen Unteransprüchen angegeben.

Die Erfindung geht aus von einer Trochanterplatte, wie sie in der WO 2004/045432 A1 angegeben ist.

Erfindungsgemäß weist die Trochanterplatte mehrere Bohrungen auf, von denen wenigstens eine der Bohrungen Mittel zur befestigungsunabhängigen Ausrichtung einer ersten Knochenschraube in einem eng begrenzten Winkelbereich aufweist. Zweite Knochenschrauben sind polyaxial einsetzbar und lassen sich mittels Schraubkappen in den Bohrungen der Trochanterplatte durch Klemmung zwischen Bohrungsrand und Schraubkappe fixieren. Die Trochanterplatte weist in ihrer Längserstreckung einen Ansatz auf bzw. ist in Längserstreckung länger als in Quererstreckung ausgebildet, wobei in dem Ansatz wenigstens eine Bohrung zur Aufnahme von dritten Schrauben mit oder ohne Winkelfixierung vorhanden ist, durch die eine oder mehrere Knochenschrauben in den Schaft des Femur einschraubbar ist. Schließlich ist die Trochanterplatte entsprechend dem Oberflächenverlauf im Bereich des proximalen Endes eines Femurknochens vorgeformt.

Mit der Erfindung wird erreicht, dass einerseits eine sichere Verbindung zwischen den Knochenfragmenten durch Verwendung von Zugschrauben herstellbar ist, die als Spongiosaschrauben ausgebildet sind und damit an die Knochensubstanz angepasst sind. Des Weiteren ist wenigstens eine winkelstabile Knochenschraube vorhanden, die entweder winkelstabil befestigbar ist oder winkelstabil in Längsrichtung verschieblich ist. Durch Anpassung der Trochanterplatte an das proximale Ende des Femurknochens wird vermieden, dass Punktbelastungen auf dem Knochen entstehen, die zu weiteren Schäden führen könnten.

Als winkelstabile Schraube kann zwar eine in einer festen Winkelrichtung in die Trochanterplatte einsetzbare Schraube verwendet werden, deren Kopf z. B. mit einem Gewinde versehen ist, das in ein entsprechendes Gewinde der Trochanterplatte einschraubbar ist. Bevorzugt ist jedoch, dass eine Verschiebemöglichkeit der winkelstabilen Schraube möglich ist, indem die Schraube in einer Führungshülse aufgenommen istt, die in eine Bohrung des Knochens eingeführt werden kann und fest mit der Trochanterplatte verbunden ist. Eine darin geführte Schraube wird axial stabil gehalten, sie ermöglicht jedoch eine Längsverschiebung.

Der Winkelbereich der Führungshülse beträgt etwa 90 - 150° zur Längsrichtung der Trochanterplatte.

Die polyaxial ausgebildeten Schrauben werden vorzugsweise durch Schraubkappen fixiert, die nach Befestigung der Schrauben auf ein Innengewinde der Trochanterplatte gesetzt werden und die Schraubköpfe derart klemmen, dass keine Winkelbewegung der Schrauben mehr möglich ist. Um zu vermeiden, dass ein selbsttätiges Lösen der Schraubkappen auftreten kann, kann die Verbindung zwischen Schraubkappe, Schraubkopf und Trochanterplatte mit einem Puffer, wie z. B. einem Federring oder einer anderen flexiblen Einlage, versehen sein oder die Schraubkappe kann ein selbsthemmendes Gewinde aufweisen, wodurch ein selbsttätiges Lösen der Befestigung des Schraubenkopfes verhindert wird. Ferner können die Schraubenköpfe eine äußere Profilierung, z. B. ein Gewinde oder eine Riffelung, aufweisen, um die Haltekraft zwischen Schraubenkopf und Schraubenkappe zu erhöhen. Um auch nach dem Einschrauben der Schraubkappen vor einer endgültigen Festlegung noch eine Nachjustierung der Schrauben zu ermöglichen, können die Schraubkappen optional zentrale Öffnungen aufweisen, durch die die Knochenschrauben mittels Werkzeug drehbar sind.

Die Trochanterplatte kann außerdem mehrere verteilte kleine Öffnungen zur Durchführung von Befestigungsdrähten enthalten. Außerdem kann die Trochanterplatte so ausgebildet sein, dass sie im Bereich der Bohrungen eine vergrößerte Dicke aufweist, um einerseits im Bereich der Bohrungen ausreichend Material zur Befestigung der Schrauben zu erreichen, aber andererseits die Dicke der Trochanterplatte im übrigen Bereich so klein wie möglich machen zu können.

In einer bevorzugten Ausgestaltung eines Systems der Erfindung weist die Trochanterplatte eine Führungshülse auf zur Aufnahme einer winkelstabilen Schraube, drei polyaxial verankerbare Spongiosaschrauben sowie wenigstens eine im Ansatz der Trochanterplatte enthaltene Schraube zur Befestigung der Trochanterplatte am Schaft des Femur.

Mit Hilfe der Erfindung lässt sich sowohl eine Kompression zwischen den Knochenfragmenten erreichen als auch eine winkelstabile Verbindung zwischen Schrauben und Trochanterplatte. Die Möglichkeit der polyaxialen Festlegung der Schrauben erleichtert die Anpassung an die individuellen anatomischen Verhältnisse. Durch ausschließliche Verwendung von Spongiosaschrauben wird erreicht, dass die Schrauben fest im Inneren der Knochen verankert sind.

Die Erfindung kombiniert das Prinzip, einerseits verschiebbare, aber winkelstabile Schrauben zu verwenden, die bei Belastung der Knochenfragmente eine Kompression ermöglichen und damit den Heilungsprozess fördern und andererseits durch einschraubbare Spongiosaschrauben eine mögliche Verkürzung des Femurhalses zu verhindern, so dass im Ergebnis die Vorteile einer dynamischen Belastungswirkung mit einer Fixierung der Fragmente erreichbar ist und damit insgesamt ein besseres Heilungsergebnis erzielt wird.

Dadurch, dass die Spongiosaschrauben nicht parallel verankert werden, wird vermieden, dass sich die Schrauben lösen können und eine Femurhalsverkürzung auftritt. Die winkelstabile Festlegung der Schrauben führt zu hohem Widerstand gegen Scher- und Torsionskräfte.

Durch die Erfindung wird erreicht, dass die Kompression zwischen den Fragmenten durch Verwendung von Zugschrauben unterstützt wird. Mit Ausnahme wenigstens einer längsverschieblichen Schraube sind alle übrigen Schrauben vorzugsweise fest und winkelstabil mit der Trochanterplatte verbunden. Indem alle Spongiosaschrauben Zugschrauben sind, lassen sich die Zugkräfte auf einen größeren Volumenbereich des Knochens verteilen.

Die Erfindung wird nachstehend anhand eines Ausführungsbeispiels näher erläutert.
- Fig. 1: zeigt eine Trochanterplatte am proximalen Ende des Femurknochens,
- Fig. 2: zeigt eine Spongiosaschraube mit einer Schraubkappe, und
- Fig. 3: zeigt eine Querschnittsansicht einer Schraube.

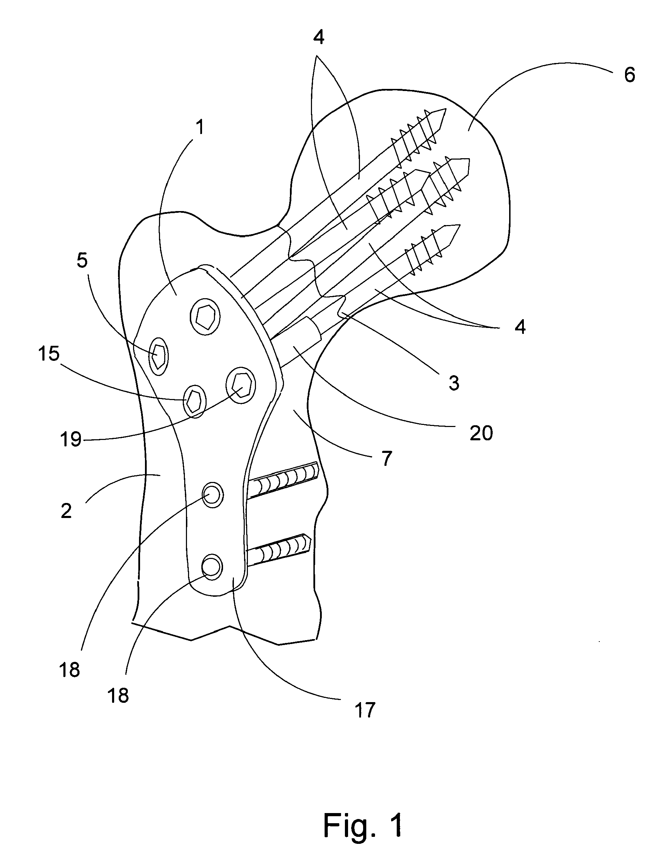

Die in Fig. 1 im Prinzip dargestellte Trochanterplatte 1 ist im Hauptteil als rechteckförmige Platte ausgebildet und der äußeren Form des Femur 2 angepasst. Es sind vier Bohrungen in der Trochanterplatte 1 dargestellt, die zweite Spongiosaschrauben 4 aufnehmen, deren Gewindeende in den Kopf 6 des Femurknochens hineinragt. Die zweiten Schrauben sind als Zugschrauben ausgeführt, so dass sie beim Festziehen über der Trochanterplatte 1 die Bruchstelle 3 des Femurknochens komprimieren. Die Mehrzahl der zweiten Schrauben 4 führt dazu, dass die Belastung sich auf eine relativ große Fläche der Trochanterplatte verteilen lässt. Da der Halsbereich 7 zwischen proximalen Ende des Femur und Kopf 6 relativ schmal ist, werden die Schrauben vorzugsweise so in die Knochenfragmente eingeschraubt, dass die Schraubenschäfte im Bereich des Oberschenkelschafts 7 des Knochens relativ eng beieinander liegen, während sie im Kopf 6 auseinandergespreizt sind, was dazu führt, dass sie auch in der Trochanterplatte relativ weit auseinander liegen. Die (zweiten) Schrauben sind damit im Wesentlichen kreuzweise übereinander oder divergierend angeordnet und ihr Verlauf im Knochen ist an die Knochenform angepasst. Die Anbringung der Schrauben in unterschiedlichen Winkeln hat auch den Vorteil, dass die Zugkräfte sich über einen größeren Winkelbereich verteilen, anders als bei einer parallelen Anordnung der Schrauben.

Die Trochanterplatte ist ferner mit einem unteren Ansatz 17 dargestellt. Darin befinden sich zwei dritte Schrauben 18, die ausschließlich im Schaft des Knochens verankert werden und vorzugsweise winkelstabil verankert sind.

Die Trochanterplatte enthält auf ihrer dem Knochen zugewandten Seite auch eine mit einer Öffnung 19 versehene Führungshülse 20, in der eine in den Knochen eingeschraubte kopflose erste Schraube geführt ist. Diese Schraube ist durch die Führungshülse 20 winkelstabil geführt, jedoch längsaxial bewegbar gehalten.

In der Praxis wird die kopflose erste Schraube, deren Gewindebereich einen größeren Durchmesser als der Schaftbereich aufweist, zunächst in einem bestimmten Winkel, der dem Winkel der Führungshülse gegenüber der Plattenebene der Trochanterplatte entspricht, in den Knochen eingeschraubt.

In einer zweiten Ausführungsform weist die Führungshülse einen Innendurchmesser auf, der minimal größer als der Durchmesser des Gewindes der ersten Schraube ist. Dadurch kann die erste Schraube nach dem Aufsetzen und Befestigen der Trochanterplatte am Knochen durch die Führungshülse in den Knochen geschraubt werden. Um gleichwohl eine gute Führung der ersten Schraube zu erreichen, weist der Schaftbereich der Schraube entweder einen Durchmesser auf, der dem Außendurchmesser des Schraubengewindes entspricht oder es wird zusätzlich eine nach dem Einschrauben der ersten Schraube in den Knochen in die Führungshülse einsetzbare Stabilisierungshülse mit einem Innendurchmesser, der dem Außendurchmesser des Schaftbereichs der Schraube und einem Außendurchmesser, der dem Innendurchmesser der Führungshülse entspricht, verwendet, die den Schaftbereich der Schraube so führt, dass keine unzulässige Winkeländerung der Schraube möglich ist.

In der ersten Ausführungsform muss die erste Knochenschraube zunächst in einem genauen Winkel in den Knochen eingeschraubt werden, der dem Winkel der Führungshülse entspricht. In der zweiten Ausführungsform wird der Einschraubwinkel selbsttätig durch den Winkel der Führungshülse an der bereits befestigten Trochanterplatte bestimmt, so dass damit Fehlanpassungen des Schraubwinkels vermieden werden können.

Die übrigen (zweiten) Schrauben 4 sind auch winkelstabil aber nicht längsaxial in der Trochanterplatte 1 verschiebbar, wenn Sie mit Schraubkappen 8 in der Trochanterplatte 1 fest geklemmt werden. Wenn keine Schraubkappen 8 verwendet werden und die Schrauben parallel zur Führungshülse 20 eingeführt werden (oder die Schrauben parallel verankert werden, wenn keine Führungshülse vorhanden ist), kann eine dynamische Kompression der Knochenfragmente (Oberschenkelkopf 6 und Oberschenkelschaft 7) an der Bruchstelle 3 erfolgen, wenn der Patient das Bein belastet. Dies kann bekanntermaßen zur Stimulierung der Zellen im Frakturspalt und damit zur verbesserten Frakturheilung führen. Der Nachteil davon ist allerdings eine mögliche Oberschenkelhalsverkürzung, die erwiesenermaßen negative Auswirkungen auf die Funktion der Hüfte hat. Bei der Verwendung der vorliegenden Erfindung kann der Chirurg entscheiden, ob er die Schrauben divergent oder konvergent einsetzt, mit Schraubenkappen versieht, und damit eine Oberschenkelhalsverkürzung verhindert, oder ob er die Schrauben parallel einsetzt, und damit eine dynamische Kompression im Frakturspalt erlaubt. Je nach Frakturtyp kann das eine oder andere vorteilhafter sein. Die Wahl zwischen diesen beiden Fixierungsprinzipien ist ein wesentlicher Vorteil dieser Erfindung im Vergleich zu bisher existierenden Systemen.

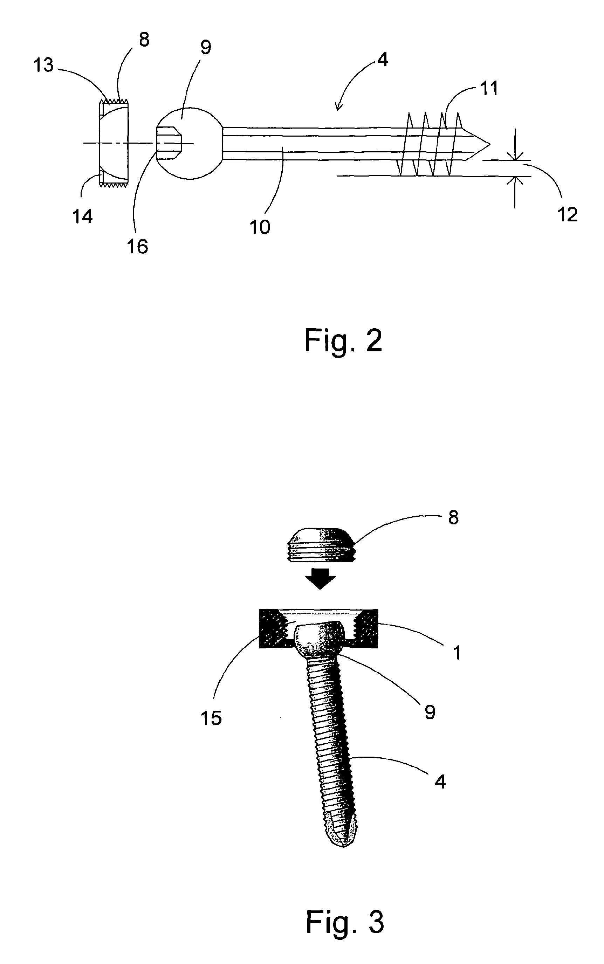

Fig. 2 zeigt eine polyaxial verwendbare Schraube mit einem Schraubenschaft 10, einem Gewinde 11 und einem Kopf 9. Die Gewindehöhe 12 beträgt etwa 1 mm, so dass eine gute Verankerung im relativ weichen Knocheninneren möglich ist. Der Schraubenkopf 9 wird in einem Hohlraum der Trochanterplatte 1 aufgenommen und kann in einem weiten Winkelbereich über eine an seiner Oberseite befindlichen Imbusöffnung 16 in den Knochen eingeschraubt werden. Durch Aufsetzen und Festziehen der Schraubenkappe 8 über das Gewinde 13 mittels der Eingriffsvertiefungen 14, die mit einem entsprechenden Werkzeug betätigt werden können, wird der Kopf 9 der Schraube in den Hohlräumen 15 der Trochanterplatte 1 fixiert und damit winkelstabil und axial fest gehalten.

Fig. 3 zeigt eine Querschnittsansicht einer Schraube 4 in der Trochanterplatte 1, bei der die Schraube durch Aufsetzen und Festziehen des Schraubkopfes 9 mittels der Schraubenkappe 8 winkelstabil gehalten werden kann.

### Bezugszeichen

- 1: Trochanterplatte
- 2: Femur
- 3: Bruchstelle
- 4: Schraube
- 5: Schraubenkopf
- 6: Oberschenkelkopf
- 7: Oberschenkelschaft
- 8: Schraubkappe
- 9: Schraubenkopf
- 10: Schraubenschaft
- 11: Gewinde
- 12: Gewindehöhe
- 13: Gewinde
- 14: Eingriffsvertiefungen
- 15: Hohlraum
- 16: Imbusöffnung
- 17: Ansatz
- 18: Schraube
- 19: Öffnung
- 20: Führungshülse

## Patentansprüche

1. Trochanterplatte zur Fixation eines Oberschenkelhalsknochens nach einer Fraktur mittels erster, zweiter und dritter Knochenschrauben, wobei die Trochanterplatte (1) Bohrungen zur Aufnahme und Winkelfixierung der Knochenschrauben (4) aufweist, **dadurch gekennzeichnet, dass** wenigstens eine der Bohrungen Mittel (20) zur befestigungsunabhängigen Ausrichtung einer ersten Knochenschraube in einem begrenzten Winkelbereich aufweist, dass die Schraubenköpfe (5) der als Spongiosaschrauben ausgebildeten zweiten Knochenschrauben (4) mittels Schraubkappen (8) in den Bohrungen der Trochanterplatte durch Klemmung gegen Bohrungsrand und Schraubkappe fixierbar sind, und dass die Trochanterplatte (1) in Längsrichtung einen Ansatz (17) aufweist, der wenigstens eine Bohrung zur Aufnahme von dritten Schrauben (18) mit oder ohne Winkelfixierung enthält, die in den Knochenschaft einschraubbar sind, wobei die Trochanterplatte (1) entsprechend dem Oberflächenverlauf im Bereich des proximalen Endes eines Femurknochens vorgeformt ist.

2. Trochanterplatte nach Anspruch 1, **dadurch gekennzeichnet, dass** das Mittel zur Ausrichtung einer ersten Knochenschraube durch eine oder mehrere in einen Knochen einführbare an der Trochanterplatte befestigte Führungshülsen (20) für den Schaft einer Knochenschraube gebildet ist

3. Trochanterplatte nach Anspruch 1, **dadurch gekennzeichnet, dass** der Winkelbereich der Führungshülsen (20) 90 - 150° zur Längsrichtung der Trochanterplatte beträgt.

4. Trochanterplatte nach Anspruch 1, **dadurch gekennzeichnet, dass** die Schraubkappen mittels selbsthemmenden Gewindes in den Bohrungen der Trochanterplatte festlegbar sind.

5. Trochanterplatte nach Anspruch 1, **dadurch gekennzeichnet, dass** die Schraubkappen (8) zentrale Öffnungen aufweisen, durch die hindurch die Knochenschrauben mittels Werkzeug drehbar sind.

6. Trochanterplatte nach Anspruch 5, **dadurch gekennzeichnet, dass** die Trochanterplatte verteilte Öffnungen zur Durchführung von Befestigungsdrähten enthält.

7. Trochanterplatte nach Anspruch 1, **dadurch gekennzeichnet, dass** die Trochanterplatte im Bereich der Bohrungen eine vergrößerte Dicke aufweist.

8. Trochanterplatte nach Anspruch 1, **dadurch gekennzeichnet, dass** die Schraubenköpfe eine äußere Profilierung zur Erhöhung der Haltekraft zwischen Schraubenkopf und Schraubenkappe aufweisen.

9. Trochanterplatte nach Anspruch 8, **dadurch gekennzeichnet, dass** die Profilierung ein Gewinde ist.

10. Trochanterplatte nach Anspruch 1, bei der die Bohrungen (18) im Ansatz der Trochanterplatte (17) zur Aufnahme von dritten Schrauben in den Knochenschaft ein Innengewinde aufweisen zur mono-axialen, winkelstabilen Aufnahme von Schrauben mit einem entsprechenden Gegengewinde am Schraubenkopf.

11. Trochanterplatte nach Anspruch 2, **dadurch gekennzeichnet, dass** die Führungshülse (20) an der Außenseite der Trochanterplatte mit einer Abdeckung abdeckbar ist.

12. System zur Fixierung eines Oberschenkelhalsknochens nach einer Fraktur mittels einer an das proximale Ende eines Femurknochens ansetzbaren Trochanterplatte (1), durch die im Winkel fixierbare erste, zweite und dritte Knochenschrauben (4) bis in den Knochenkopf (6) einschraubbar sind, **dadurch gekennzeichnet, dass** die Trochanterplatte (1) wenigstens eine zum Knochen gerichtete Führungshülse (20) zur Führung des Schaftes einer ersten Knochenschraube aufweist, wobei die Führungshülse in das an die Trochanterplatte angrenzende Knochenteil einführbar ist, dass die Knochenschrauben (4) als Zugschrauben ausgeführte Spongiosaschrauben sind, und dass die Schraubenköpfe (5) der zweiten Knochenschrauben in der Trochanterplatte in einem festen Winkel zur Längsachse der Trochanterplatte fixierbar sind und die wenigstens eine erste Schraube, die in der Führungshülse aufgenommen ist, in Achsrichtung der Schraube über eine begrenzte Strecke in der Trochanterplatte verschiebbar gehalten ist.

13. System nach Anspruch 12, **dadurch gekennzeichnet, dass** von vier eingesetzten Knochenschrauben drei zweite Knochenschrauben als fixierte Schrauben und eine erste Knochenschraube als in einer Führungshülse (20) längsverschiebliche Schraube ausgebildet sind.
